# EUROPÄISCHE PATENTSCHRIFT

(11) **EP 2 030 597 B1**
(45) Veröffentlichungstag und Bekanntmachung des Hinweises auf die Patenterteilung: **21.09.2011**
(21) Anmeldenummer: 08013144.4
(22) Anmeldetag: 22.07.2008
(51) Int. Cl.: A61F 2/46

(54) **Vorrichtung und Verfahren zum Mischen von Knochenzement**
Method and device for mixing bone cement
Dispositif et procédé destinés au mélange de ciment osseux

(30) Priorität: 01.09.2007 DE 102007041666
(43) Veröffentlichungstag der Anmeldung: 04.03.2009
(73) Patentinhaber: aap Biomaterials GmbH & Co. KG, 64807 Dieburg (DE)
(72) Erfinder: Deußer, Stefan, 63791 Karlstein (DE); Sattig, Christoph, 64807 Dieburg (DE)
(74) Vertreter: Herden, Andreas F.

(56) Entgegenhaltungen:
- EP-A- 0 178 658
- US-A- 4 185 072
- US-A- 5 462 356
- US-A- 5 549 381

## Beschreibung

### Gebiet der Erfindung

Die Erfindung bezieht sich auf eine Vorrichtung und ein Verfahren zum Mischen von Pasten, insbesondere Knochenzementmischung zur Injektion in die Wirbelkörper.

### Hintergrund der Erfindung

Im Rahmen von Vertebroplastie- und Kyphoplastiebehandlungen werden angemischte Pasten zur Auffüllung und Stabilisierung der Wirbelkörper, z.B. bei frischen Frakturen, verwendet.

Der hierfür notwendige Knochenzement besteht entweder aus Hydroxylapatit, einem Calciumphospahtsalz, oder aus Acrylaten. Beiden verwendeten Arten ist gemeinsam, dass sie im Operationssaal unmittelbar vor dem Eingriff aus zwei Komponenten, einer festen und einer flüssigen, angemischt werden und in situ aushärten.

Zur Anmischung des Knochenzements sind verschiedene Vorrichtungen und Verfahren bekannt. Die gattungsbildende US 5,549,381 zeigt eine Vorrichtung zum Anmischen von Knochenzement mit einem kreiszylinderförmigen Mischbehälter. Die EP 0 178 658 A2 beschreibt beispielsweise eine Vorrichtung und ein Verfahren zur Herstellung von Knochenzement zwecks Fixierung von Prothesen, wie künstlichen Hüftgelenken. Die Vorrichtung umfasst ein Gefäß, in dem der Knochenzement mittels einer motorischen Rührvorrichtung angemischt wird. Das heftige Rühren führt zu Lufteinschlüssen in der Mischung des Knochenzeinents, die entfernt werden müssen. Hierzu wird eine Saugpumpe an das Mischgefäß angeschlossen, die während des Mischvorgangs im Inneren des Gefäßes ein Vakuum erzeugt. Bei der Entnahme des Knochenzements wird verhindert, dass Luft in die Entnahmespritze gelangt.

Rührwerk und notwendige Pumpe sind kostenintensiv. Nachteilig ist ferner, dass mit der bekannten Vorrichtung nur größere Mengen an Knochenzement angemischt werden können.

### Allgemeine Beschreibung der Erfindung

Die Aufgabe der Erfindung besteht darin, eine einfache und kostengünstige Vorrichtung zur Mischung von Knochenzement bereit zu stellen.

Eine weitere Aufgabe der Erfindung besteht darin, den angemischten Knochenzement in einer kleineren Menge bereit zustellen, wie sie für die Injektion in Wirbelkörper benötigt wird.

Ferner liegt der Erfindung die Aufgabe zugrunde, zu verhindern, dass während der Entnahme des angemischten Knochenzements Luft in die Entnahmevorrichtung gelangt.

Zur Lösung dieser Aufgaben wird ein Mischgefäß verwendet, in dem aus zwei Komponenten manuell mit Hilfe eines Handrührstabes eine kleinere Menge an Knochenzement angemischt werden kann.

Das Mischgefäß aus Kunststoff ist innen von nichteckiger Form, so dass keine Mischungsanteile in toten Winkeln verbleiben, wodurch eine Verfälschung des Mischungsverhältnisses im nutzbaren Volumen des Mischbechers vermieden wird. Außerdem können nur geringe Knochenzementreste im Inneren des Gefäßes zurück bleiben.

Sobald der Knochenzement angemischt ist, wird ein Deckel in das Mischgefäß eingesetzt. Anschließend wird der Deckel durch Herabdrücken im Mischgefäß abgesenkt. Die ursprünglich zwischen Knochenzement und Deckel befindliche Luft wird hierbei zwischen den Seitenwänden des Gefäßes und des Deckels als auch durch die zentrale Öffnung herausgepresst. Schließlich sitzt der Deckel auf dem angemischten Knochenzement auf, so dass sich oberhalb des Zements keinerlei Luft mehr befindet. Nach dem Herabsenken des Deckels ist also das Mischgefäß mit dem darin befindlichen angemischten Knochenzement luftdicht abgeschlossen oder "versiegelt".

Zwecks Entnahme des angemischten Knochenzements befindet sich am unteren Ende des Deckels eine kegelförmige Entnahmevorrichtung. Diese Entnahmevorrichtung ist dergestalt konstruiert, dass nur durch sie eine Entnahme ermöglicht wird. Durch den geringen Querschnitt wird eine Belastung des OP-Personals durch Monomerdämpfe minimiert. Bei dieser Entnahmevorrichtung handelt es sich vorzugsweise um einen Luer-Kegel.

In diese Entnahmevorrichtung werden eine oder nacheinander mehrere Entnahmespritzen eingesetzt, mit welcher der angemischte Knochenzement aus dem Mischgefäß abgesaugt werden kann. Während des Absaugvorgangs drückt der äußere Luftdruck den Deckel entsprechend der abgesaugten Menge an Knochenzement herab, wobei der Spalt zwischen Mischgefäß und Deckel durch die plastische Knochenzementmischung abgedichtet gehalten wird. Somit wird verhindert, dass während des Entnahmevorgangs Luft in die Mischung und damit in die Entnahmespritze gelangen kann.

Sobald die gewünschte Menge an Knochenzement entnommen wurde, wird die Entnahmespritze entfernt.

In einer Ausführungsform der Erfindung wird das Mischgefäß als Liefergefäß der einen Komponente des anzumischenden Knochenzements, vorzugsweise der festen Komponente, verwendet. Hierzu wird das Mischgefäß mit der darin befindlichen Komponente mittels Abdeckfolie verschlossen, und zwar wird vorzugsweise die Abdeckfolie an einem Flansch befestigt, der sich am oberen Rand des Mischgefäßes befindet. In dieser Form kann das Mischgefäß zusammen mit z.B. einem Handrührstab, dem Deckel und der zweiten Komponente des später anzumischenden Knochenzements als "Set" gewerblich vertrieben werden.

### Kurzbeschreibung der Figuren

Ein Ausführungsbeispiel der Erfindung wird anhand der Zeichnungen beschrieben.

Dabei zeigen:
Fig.1a einen Vertikalschnitt eines erfindungsgemäßen Mischbechers gemäß Schnitt AA in Fig. 1c,
Fig. 1b den Mischbecher im Vertikalschnitt gemäß Schnitt BB in Fig. 1c
Fig. 1c den Mischbecher in einer Draufsicht,
Fig. 2a einen Vertikalschnitt eines Deckels des Mischbechers gemäß Schnitt AA in Fig. 2c,
Fig. 2b den Deckel im Vertikalschnitt gemäß Schnitt BB in Fig. 2c
Fig. 2c den Deckel in einer Draufsicht,
Fig. 3a den Deckel in eingesetztem, nicht abgesenkten Zustand, und
Fig. 3b einen Vertikalschnitt des Deckels im abgesenkten Zustand.

### Detaillierte Beschreibung der Erfindung

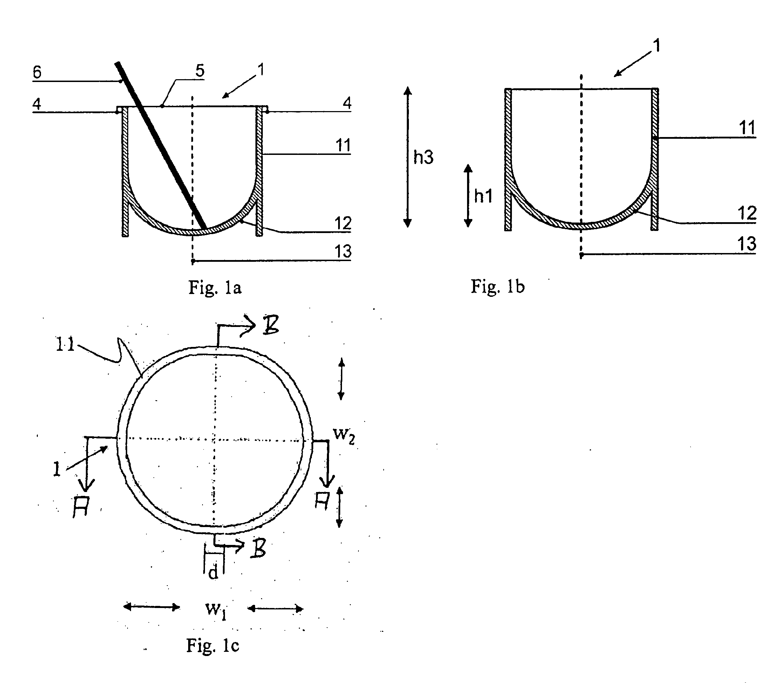

Fig. 1a und 1b zeigen den erfindungsgemäßen Mischbecher 1 in je einem vertikalen Schnitt. Der Mischbecher 1 hat einen Boden 12 von gewölbter Form. An diesen Boden 12 schließt sich eine Seitenwand 11 an, die nach unten Füße bildet und nach oben hochgezogen ist.

Am oberen Ende der Seitenwand 11 kann ein Flansch 4 verlaufen, an dem eine Abdeckfolie 5 befestigt werden kann.

Die Gesamtkonfiguration des Mischbechers 1 weist eine Symmetrieachse 13 auf. Im axialen Schnitt erscheint die Wölbung des Bodens 12 als angenähertes halbes Ellipsoid. Die hochgezogene Seitenwand verläuft parallel zur Symmetrieachse 13.

Der Boden 12 weist eine Höhe h1 von 22 mm ± 50% auf, woran sich die Seitenwand über eine Strecke h2 von 20 mm ± 50% anschließt, so dass sich eine Gesamthöhe h3 des Mischbechers von 42 mm ± 50%ergibt.

Die Fig. 1c zeigt den Mischbecher 1 in einer Draufsicht. Die Seitenwand 11 weist einen eckenlosen Querschnitt auf, ohne kreisförmig zu sein. Die Seitenwand 11 bildet in einer bevorzugten Ausführungsform der Erfindung einen angenäherten elliptischen Zylinder, der in seiner größten Ausdehnungsrichtung eine innere Weite w₁ von 49 mm ± 50% aufweist. Senkrecht hierzu beträgt die innere Weite w₂ der angenäherten Ellipse 44 mm ± 50%. Die Differenz zwischen diesen beiden Werten ist darin begründet, dass in der größten Ausdehnungsrichtung die Seitenwand 11 zwei einander gegenüber liegende lineare Abschnitte von 5 mm ± 50% aufweist.

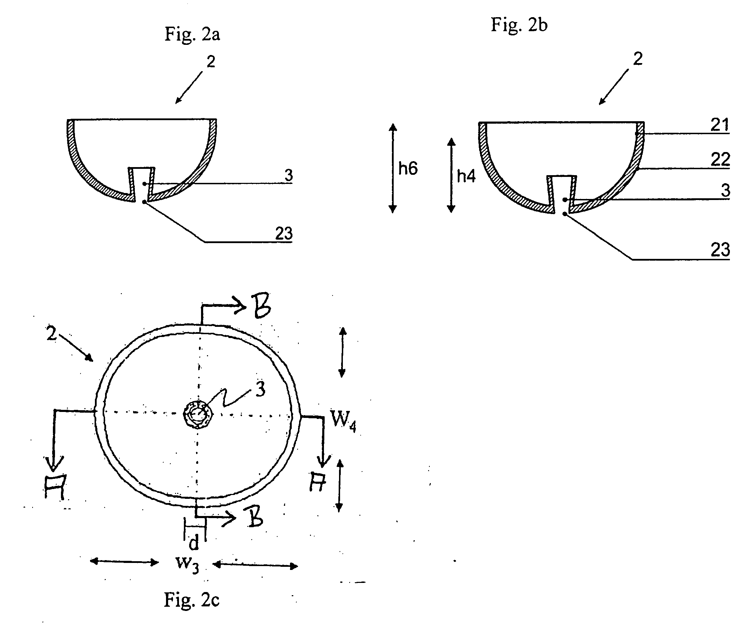

Fig. 2a zeigt den Deckel 2 der erfindungsgemäßen Vorrichtung in einem vertikalen Schnitt. An der untersten Stelle des Deckels 2 befindet sich ein Entnahmehohlkegel 3, der die Wandung des Bodens 22 durchstößt. Bei diesem Entnahmehohlkegel 3 handelt es sich vorzugsweise um einen 6% Luer-Kegel nach ISO 594-1. Hinsichtlich der Geometrie des Entnahmehohlkegels 3 bedeutet dies, dass die Seitenwand des Entnahmehohlkegels 3 eine 6%ige Steigung aufweist.

Beim Luer-Kegel handelt es sich um ein genormtes Verbindungssystem für Kanülen, Spritzen und Infusionsschläuche im medizinischen Bereich. Die Dichtung, bei der Erfindung zwischen Entnahmehohlkegel 3 und Entnahmespritze, wird durch eine kegelförmige Konstruktion der Verbindungsteile erzielt.

Der Entnahmehohlkegel 3 ist zur Fixierung einer Entnahmespritze (nicht abgebildet) ausgebildet, mit welcher angemischter Knochenzement aus dem Mischbecher 1 abgesaugt werden kann.

Fig. 2b zeigt in einem vertikalen Schnitt den Deckel 2 der erfindungsgemäßen Vorrichtung. Bei jeder Ausführungsform ist die äußere Weite der Seitenwand 21 des Deckels 2 gleich der inneren Weite der Seitenwand 11 des Mischbechers 1, um sicherzustellen, dass der Deckel 2 passgenau in den Mischbecher 1 passt.

Der gewölbte Boden 22 des Deckels 2 erstreckt sich bis in eine Höhe h4 von 22 mm ± 50% vom untersten Punkt des Deckels 2 aus gemessen. An den gewölbten Boden 22 schließt sich eine hoch gezogene Seitenwand 21 mit einer Höhe h5 von 3 mm ± 50% an, so dass sich für die Gesamthöhe h6 des Deckels 2 der erfindungsgemäßen Vorrichtung ein Wert von 25 mm ± 50% ergibt.

Fig. 2c zeigt die Abmessungen des Deckels der erfindungsgemäßen Vorrichtung in einer Draufsicht. Die Seitenwand 21 bildet eine angenäherte Ellipse, welche in ihrer größten Ausdehnungsrichtung eine äußere Weite w₃ von 49 mm ± 50% aufweist. Senkrecht hierzu beträgt die äußere Weite w₄ der angenäherten Ellipse 44 mm ± 50%. Die Differenz zwischen diesen beiden Werten ist darin begründet, dass in der größten Ausdehnungsrichtung die Seitenwand 21 zwei einander gegenüber liegende lineare Abschnitte von 5 mm ± 50% aufweist. Mit dieser Ausbildung wird vermieden, dass sich der Deckel beim Befestigen und Entfernen des Entnahmesystems verdreht.

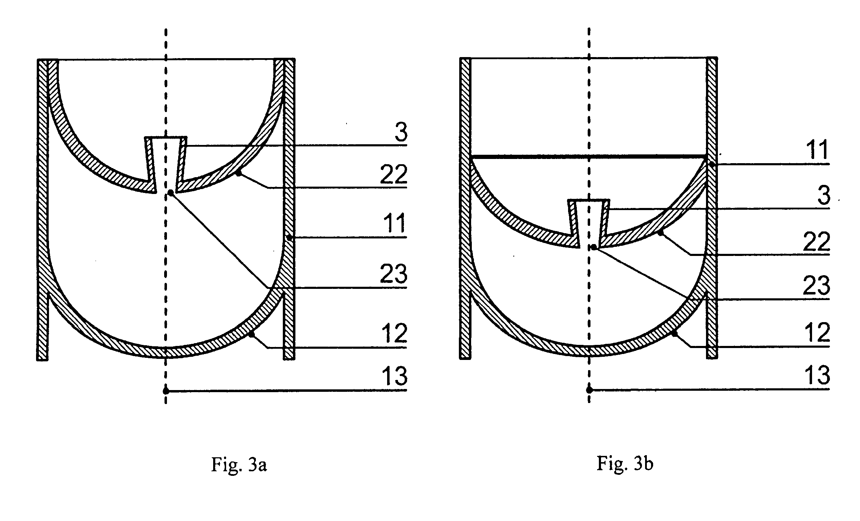

Fig. 3a zeigt in einem vertikalen Schnitt den erfindungsgemäßen Mischbecher 1 mit eingesetztem Deckel 2, wobei sich der Deckel 2 passgenau im oberen Bereich des Mischbechers 1 befindet.

Fig. 3b zeigt in einem vertikalen Schnitt den erfindungsgemäßen Mischbecher 1 mit eingesetztem Deckel 2, wobei der Deckel 2 soweit abgesenkt worden ist, dass sich nur noch Knochenzementmischung im Gefäßinneren befindet, d.h. Luft, die sich zunächst zwischen Mischungsoberfläche und Deckel befunden hat, ist durch den abgesenkten Deckel seitlich über die Lücke zwischen Gefäßwand 11 und Deckel 2 und der Entnahmeöffnung verdrängt worden. In diesem Zustand ist die Mischung durch den Deckel fast komplett abgeschlossen. Lediglich der Querschnitt der zentralen Öffnung steht mit der umgebenden Luft in Verbindung.

Die Handhabung der Mischvorrichtung ist wie folgt:
Am Boden des Mischbechers 1 kann mittels eines Handrührstabes 6 Knochenzement manuell angerührt werden. Der Knochenzement wird aus zwei Komponenten hergestellt, einer festen, evtl. pulvrigen und einer flüssigen. Da der Boden 12 eine abgerundete Gestalt aufweist, können sich an keiner Stelle des Bodens 12 Komponenten der Mischung oder Reste festsetzen.

Nachdem im Mischbecher 1 manuell mittels des Handrührstabs 6 aus zwei Komponenten Knochenzement angemischt worden ist, wird der Deckel 2 in den Mischbecher 1 eingesetzt (Fig. 3a). Der so eingesetzte Deckel 2 wird im Mischbecher 1 herabgedrückt, um die oberhalb des angemischten Knochenzements befindliche Luft aus dem Mischbecher 1 seitlich und durch die zentrale Öffnung zu verdrängen. Im nächsten Schritt wird das Mündungsstück einer Entnahmespritze in den Entnahmehohlkegel 3 eingeführt. Mittels der derart eingesetzten Entnahmespritze wird nunmehr der angemischte Knochenzement aus dem Mischbecher 1 abgesaugt. Während dieses Absaugvorgangs senkt sich der Deckel 2 entsprechend der entnommenen Menge an angemischtem Knochenzement ab, da der Deckel 2 passgenau im Mischbecher 1 sitzt und der angemischte Knochenzement als Dichtmittel wirkt. Somit ist der Bereich des Mischbechers 1, in dem sich der angemischte Knochenzement befindet, gegenüber der Außenwelt luftdicht abgeschlossen. Daher verursacht der Luftdruck außerhalb des Mischbechers 1 mit eingesetztem Deckel 2 das Absenken des Deckels 2 während des Entnahmevorgangs. Sobald sich die gewünschte Menge an angemischtem Knochenzement in der Entnahmespritze befindet, wird diese aus dem Entnahmehohlkegel abgezogen.

Bei dem angemischten Knochenzement handelt es sich vorzugsweise um Hydroxylapatit in Pulverform, mit einem Bindemittel vermischt. Nach Aushärtung entsteht ein Calciumphosphatsalz von hohem Härtegrad. Es kann auch Acrylpaste erzeugt werden.

Therapeutisch wird der angemischte Knochenzement vorzugsweise im Rahmen der Kyphoplastie oder der Vertebroplastie zur Injektion in zu behandelnde Wirbelkörper eingesetzt.

Allgemein kann die Erfindung im Rahmen von therapeutischen Verfahren eingesetzt werden, bei denen keine großen Mengen an angemischtem Knochenzement benötigt werden. Somit kann mit der Erfindung Zement angemischt und in Applikationssysteme transferiert werden.

Die Erfindung kann auch dergestalt gewerblich vertrieben werden, dass der Mischbecher 1 bereits ab Werk eine der beiden Komponenten des Knochenzements, vorzugsweise die feste, enthält. Der Mischbecher 1 wird dann nach Befüllen mit der einen Komponente des anzumischenden Knochenzements mittels einer am Flansch 4 befestigten Abdeckfolie 5 steril verschlossen, und so ausgeliefert.

In dieser Ausführungsform wird der Mischbecher 1 zusammen mit Deckel 2 und Handrührstab 6 in einem sterilen "Set" gewerblich angeboten.

## Patentansprüche

1. Vorrichtung zum Mischen von Knochenzement, insbesondere zur Injektion in Wirbelkörper, umfassend
einen Mischbecher (1) und
einen Deckel (2);
der Mischbecher (1) hat einen gewölbten Boden und eine hochgezogene Seitenwand (11) mit einem in der Draufsicht auf den Mischbecher eckenlosen Querschnitt, **dadurch gekennzeichnet, daß** der Querschnitt nicht kreisförmig ist,
daß der Deckel (2) eine Form aufweist, die in den Boden passgenau hineinpasstt, und daß
ferner an unterster Stelle des Deckels (2) ein Entnahmehohlkegel (3) angebrachter ist, der mit seinem Inneren die Bodenwandung des Deckels durchstößt und zur Fixierung einer Entnahmespritze während des Absaugens des angemischten Knochenzements ausgebildet ist.

2. Vorrichtung nach Anspruch 1, **dadurch gekennzeichnet, dass** der Querschnitt der hochgezogenen Seitenwand (11) des Mischbechers (1) eine angenäherte Ellipse bildet.

3. Vorrichtung nach Anspruch 1 oder 2, **dadurch gekennzeichnet, dass** der Boden des Mischbechers (1) eine Symmetrieachse (13) aufweist und die Wölbung des Bodens im axialen Schnitt durch die Symmetrieachse kreisförmig erscheint.

4. Vorrichtung nach einem der Ansprüche 1 - 3, **dadurch gekennzeichnet, dass** die hochgezogene Seitenwand (11) des Mischbechers (1) eine Symmetrieachse (13) aufweist und achsenparallel verläuft.

5. Vorrichtung nach einem der Ansprüche 1 - 4, **dadurch gekennzeichnet, dass** der Öffnungswinkel des Entnahmehohlkegels (3) eine 6%ige Steigung der Seitenwand des Kegels aufweist.

6. Vorrichtung nach einem der Ansprüche 1 - 5, **dadurch gekennzeichnet, dass** an der hochgezogenen Seitenwand (11) des Mischbechers (1) ein Flansch (4) zum Anbringen einer Abdeckfolie (5) vorgesehen ist.

7. Vorrichtung nach einem der Ansprüche 2 - 6, **dadurch gekennzeichnet, dass** der Innenraum des Mischbechers (1) eine Weite im Bereich von 44 auf 49 mm ± 50% und eine Höhe im Bereich von 42 mm ± 50 % aufweist.

8. Verfahren zur Mischung von Knochenzement mit einer Vorrichtung nach einem der Ansprüche 1 - 7, insbesondere zur Injektion in Wirbelkörper, die folgenden Schritte umfassend:
a) Anmischen von Hand des Knochenzements im Mischbecher (1) mittels eines Handrührstabs (6),
b) Einsetzen des Deckels (2) und Verdrängen der Luft oberhalb des angemischten Knochenzements im Mischbecher (1), wobei der Deckel (2) in seiner Mitte einen Entnahmehohlkegel (3) aufweist, über den der im Mischbecher (1) angemischte Knochenzement entnommen werden kann,
c) Einführen eines Mündungsstückes einer Entnahmespritze in den Entnahmehohlkegel (3),
d) Absaugen des angemischten Knochenzements mittels der Entnahmespritze aus dem Mischbecher (1) ohne ein Verdrehen desselben, wobei sich der Deckel (2) entsprechend der entnommenen Menge an angemischtem Knochenzement absenkt, und
e) Abziehen der Entnahmespritze aus dem Entnahmehohlkegel (3).

9. Verfahren nach Anspruch 8, **dadurch gekennzeichnet, dass** als angemischter Knochenzement Polymethylmetacrylat verwendet wird.

10. Verfahren nach Anspruch 8, **dadurch gekennzeichnet, dass** als angemischter Knochenzement Calciumphosphate oder Calciumsulfate verwendet werden.

11. Verfahren nach Anspruch 8 in Verbindung mit Anspruch 6, **dadurch gekennzeichnet, dass** der Knochenzement aus zwei Komponenten angemischt wird, und die eine dieser zwei Komponenten im Mischbecher (1), der mittels einer am Flansch (4) befestigten Abdeckfolie (5) geschlossen ist, zur Verfügung gestellt wird.

## Claims

1. Device for mixing bone cement, in particular for injection into vertebral bodies, comprising:
a mixing beaker (1); and
a cover (2);
the mixing beaker (1) has a curved bottom and a raised side wall (11) having a cross-section which is comer-less in the plan view of the mixing beaker, **characterised in that** the cross-section is not circular;
the cover (2) has a shape which fits into the bottom in an accurately fitting manner; and
provided at the lowermost point of the cover (2) is also an extraction hollow cone (3), the interior of which pierces the bottom wall of the cover and is designed for securing an extraction syringe during extraction of the mixed bone cement by means of suction.

2. Device as claimed in claim 1, **characterised in that** the cross-section of the raised side wall (11) of the mixing beaker (1) forms an approximated ellipse.

3. Device as claimed in claim 1 or 2, **characterised in that** the bottom of the mixing beaker (1) comprises an axis of symmetry (13), and the curvature of the bottom appears to be circular in axial cross-section through the axis of symmetry.

4. Device as claimed in any one of claims 1 - 3, **characterised in that** the raised side wall (11) of the mixing beaker (1) comprises an axis of symmetry (13) and extends in parallel with the axis.

5. Device as claimed in any one of claims 1 - 4, **characterised in that** the opening angle of the extraction hollow cone (3) comprises a 6% gradient of the side wall of the cone.

6. Device as claimed in any one of claims 1 - 5, **characterised in that** provided on the raised side wall (11) of the mixing beaker (1) is a flange (4) for attaching a cover film (5).

7. Device as claimed in any one of claims 2 - 6, **characterised in that** the interior space of the mixing beaker (1) comprises a width in the range of from 44 to 49 mm ± 50% and a height in the region of 42 mm ± 50%.

8. Method of mixing bone cement by means of a device as claimed in any one of claims 1 - 7, in particular for injection into vertebral bodies, comprising the steps of:
a) manually mixing the bone cement in the mixing beaker (1) by means of a manual stirring rod (6),
b) inserting the cover (2) and displacing air above the mixed bone cement in the mixing beaker (1), wherein the centre of the cover (2) comprises an extraction hollow cone (3), by means of which the bone cement mixed in the mixing beaker (1) can be extracted,
c) introducing a nozzle of an extraction syringe into the extraction hollow cone (3),
d) extracting by suction the mixed bone cement by means of the extraction syringe from the mixing beaker (1) without twisting same, wherein the cover (2) is lowered corresponding to the extracted quantity of mixed bone cement, and
e) removing the extraction syringe from the extraction hollow cone (3).

9. Method as claimed in claim 8, **characterised in that** polymethyl methacrylate is used as the mixed bone cement.

10. Method as claimed in claim 8, **characterised in that** calcium phosphates or calcium sulphates are used as the mixed bone cement.

11. Method as claimed in claim 8 in conjunction with claim 6, **characterised in that** the bone cement is mixed from two components, and one of these two components is provided in the mixing beaker (1) which is closed by means of a cover film (5) attached to the flange (4).

## Revendications

1. Dispositif destiné à mélanger du ciment osseux, en particulier pour une injection dans le corps vertébral, comprenant
un gobelet mélangeur (1) et
un couvercle (2) ;
le gobelet mélangeur (1) a un fond incurvé et une paroi latérale (11) relevée avec une section transversale sans angle en vue de dessus du gobelet mélangeur, **caractérisé en ce que** la section transversale n'a pas une forme circulaire,
**en ce que** le couvercle (2) présente une forme qui s'adapte de façon précise dans le fond, et
**en ce qu'**un cône creux de prélèvement (3) est placé en outre à l'endroit le plus bas du couvercle (2), lequel cône traverse avec son intérieur la paroi de fond du couvercle et est conçu pour la fixation d'une seringue de prélèvement pendant l'aspiration du ciment osseux mélangé.

2. Dispositif selon la revendication 1, **caractérisé en ce que** la section transversale de la paroi latérale (11) relevée du gobelet mélangeur (1) forme une ellipse approchée.

3. Dispositif selon la revendication 1 ou 2, **caractérisé en ce que** le fond du gobelet mélangeur (1) présente un axe de symétrie (13) et la courbure du fond paraît circulaire dans la coupe axiale dans l'axe de symétrie.

4. Dispositif selon l'une quelconque des revendications 1 à 3, **caractérisé en ce que** la paroi latérale (11) relevée du gobelet mélangeur (1) présente un axe de symétrie (13) et est agencée parallèlement à l'axe.

5. Dispositif selon l'une quelconque des revendications 1 à 4, **caractérisé en ce que** l'angle d'ouverture du cône creux de prélèvement (3) présente une pente à 6 % de la paroi latérale du cône.

6. Dispositif selon l'une quelconque des revendications 1 à 5, **caractérisé en ce qu'**une bride (4) est prévue sur la paroi latérale (11) relevée du gobelet mélangeur (1) pour la mise en place d'un film de recouvrement (5).

7. Dispositif selon l'une quelconque des revendications 2 à 6, **caractérisé en ce que** l'espace intérieur du gobelet mélangeur (1) présente une largeur de l'ordre de 44 à 49 mm ± 50 et une hauteur de l'ordre de 42 mm ± 50 %.

8. Procédé pour mélanger du ciment osseux avec un dispositif selon l'une quelconque des revendications 1 à 7, en particulier pour l'injection dans le corps vertébral, qui comprend les étapes suivantes :
a) mélange manuel du ciment osseux dans le gobelet mélangeur (1) au moyen d'un agitateur manuel (6),
b) mise en place du couvercle (2) et refoulement de l'air au-dessus du ciment osseux mélangé dans le gobelet mélangeur (1), le couvercle (2) présentant en son centre un cône creux de prélèvement (3), par lequel le ciment osseux mélangé dans le gobelet mélangeur (1) peut être prélevé,
c) introduction d'une pièce d'embouchure d'une seringue de prélèvement dans le cône creux de prélèvement (3),
d) aspiration du ciment osseux mélangé au moyen de la seringue de prélèvement hors du gobelet mélangeur (1) sans une rotation du gobelet, le couvercle (2) s'abaissant en fonction de la quantité prélevée de ciment osseux mélangé, et
e) enlèvement de la seringue de prélèvement du cône creux de prélèvement (3).

9. Procédé selon la revendication 8, **caractérisé en ce qu'**on utilise du polyméthylméthacrylate comme ciment osseux mélangé.

10. Procédé selon la revendication 8, **caractérisé en ce qu'**on utilise des phosphates de calcium ou des sulfates de calcium comme ciment osseux mélangé.

11. Procédé selon la revendication 8, en liaison avec la revendication 6, **caractérisé en ce que** le ciment osseux est mélangé à partir de deux composants et l'un de ces deux composants est mis à disposition dans le gobelet mélangeur (1), qui est fermé au moyen d'un film de recouvrement (5) fixé sur la bride (4).
